# EUROPEAN PATENT SPECIFICATION

(11) **EP 0 285 395 B1**
(45) Date of publication and mention of the grant of the patent: **11.05.1994**
(21) Application number: 88302834.2
(22) Date of filing: 30.03.1988
(51) Int. Cl.: C07D 239/24, C09K 19/34

(54) **Phenyl-pyrimidine liquid crystal compounds and liquid crystal compositions containing the same**
Phenylpyrimidin-Flüssigkristall-Verbindungen und sie enthaltende Flüssigkristall-Zusammensetzungen
Composés de phényl-pyrimidines cristaux liquides et compositions de cristaux liquides les contenant

(30) Priority: 31.03.1987 JP 78289/87; 29.10.1987 JP 274389/87
(43) Date of publication of application: 05.10.1988
(73) Proprietor: AJINOMOTO CO., INC., Tokyo 104 (JP)
(72) Inventor: Higuchi, Ryoichi, Centr.Res.Lab. Ajinomoto Co.Inc., Kawasaki-shi, Kanagawa-ken (JP); Sakurai, Takao, Centr.Res.Lab. Ajinomoto Co.Inc., Kawasaki-shi, Kanagawa-ken (JP); Yokota, Tadahiko, Centr.Res.Lab. Ajinomoto Co.Inc., Kawasaki-shi, Kanagawa-ken (JP); Mikami, Naoko, Centr.Res.Lab. Ajinomoto Co.Inc., Kawasaki-shi, Kanagawa-ken (JP); Yamamoto, Eri, Centr.Res.Lab. Ajinomoto Co.Inc., Kawasaki-shi, Kanagawa-ken (JP); Takeuchi, Koji, Centr.Res.Lab. Ajinomoto Co.Inc., Kawasaki-shi, Kanagawa-ken (JP)
(74) Representative: Bond, Bentley George

(56) References cited:
- EP-A- 0 225 195
- EP-A- 0 247 804
- GB-A- 2 049 692

## Description

The present invention relates to novel liquid crystal compounds and liquid crystal compositions containing such liquid crystal compounds.

Display devices consisting of liquid crystal display elements now widely practically used are those of twisted nematic type (TN type) and dynamic scattering type (DS type). These display devices contain nematic liquid crystal cells which in turn contain nematic liquid crystals as a main component. One of the drawbacks of the usual nematic liquid crystal cells is that the speed of response is so slow that only a response speed of the order several milliseconds is obtained at most, and this drawback makes the applicable range of the nematic crystal cells rather narrow. However, it has lately come to be recognized that the use of the smectite liquid cells makes a higher speed response possible.

Some optically active smectite liquid crystals exhibit strong dielectric properties, and the use thereof is expected to increase. Examples of liquid crystals exhibiting strong dielectric properties, namely strongly dielectric liquid crystals, are the compounds represented by 4-(4-n-decyloxybenzylideneamino) cinnamic acid-2-methyl butyl ester (hereinafter abbreviated as DOBAMBC) as synthesized by R.B. Meyer at al in 1975. These compounds are characterised by exhibiting strong dielectric properties in the chiral smectic C phase (J. Physique, 36, L-69 (1975).

Recently, a high response speed of the microsecond order has been found in a thin film cell of DOBAMBC of N.A. Clark et al. (Appl. Phys. Lett. 36, 89 (1980)). With this as a turning point, attention has been paid to strongly dielectric liquid crystals owing to their high response speed, as a material usable not only for displays such as liquid crystal televisions but also as a material for optoelectronic devices such as optical printer heads, optical Fourier transforming devices and light valves.

Display devices containing strongly dielectric liquid crystals are able to produce a display by the application of short pulses and to retain the display, and thus make it possible readily to obtain a large area of display.

However, display devices containing strongly dielectric crystal compositions have not so far been made practicable for the reason that the response speed of the composition is inadequate and that the usable temperature range is narrow.

It is primary object of the present invention to provide novel smectic phenyl-pyrimidine liquid crystal compounds and to chiral smectic C liquid crystal compositions containing the same.

In one aspect of the present invention, there is provided smectic phenyl-pyrimidine liquid crystal compounds represented by the following formula (I) 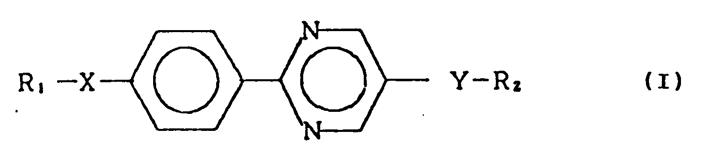
wherein R₁ and R₂ each represent an alkyl group having 1 to 18 carbon atoms, and X and Y each represent a single bond, -O-, -OCO- or -OCOO-, provided that one of X and Y is -OCOO-.

In another aspect of the present invention there is provided a strongly dielectric chiral smectic C liquid crystal composition having a high response speed over a wide range of temperature, comprising a smectic compound(s) of this invention and a chiral smectic C liquid crystal compound, or a smectic compound(s) of this invention and a chiral smectic C liquid crystal composition.

The single Figure of the drawings shows the infrared absorption spectrum, by the Nujol method, of 2-(4-decyloxyphenyl)-5-octyloxycarbonyloxypyrimidine as obtained in Example 1 hereinbelow.

The present inventors have searched for various liquid crystal compounds in order to solve the above problems and attained the present invention.

That is, the present invention relates to compounds represented by the general formula (I) above, and liquid crystal compositions containing the same.

Compounds of the general formula (I) may be synthesized as follows.

A 5-hydroxypyrimidine derivative represented by the general formula (a): 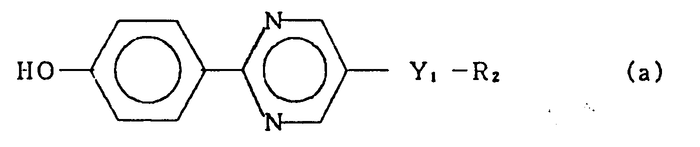
(wherein R₂ represents an alkyl group having 1 to 18 carbon atoms, and Y₁ represents a single bond, -O-, -OCO-, or -COO-) or a 5-hydroxypyrimidine derivative represented by the formula (b): 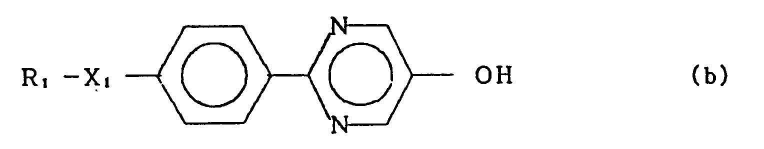
(wherein R₁ represents an alkyl group having 1 to 18 carbon atoms, X₁ represents a single bond, -O-, -OCO-or -COO-) is reacted with a chlorocarboxylic acid alkyl ester represented by the general formula (c)

R₃-OCOCl (c)

(wherein R₃ represents an alkyl group having 1 to 18 carbon atoms) in the presence of a base such as pyridine or triethylamine in an inert solvent such as carbon tetrachloride or chloroform, followed by purification to obtain a desired product.

A strongly dielectric liquid crystal composition having a high response speed over a wide range of temperature may be obtained by mixing one or more compounds of the present invention with one or more chiral smectic C liquid crystal compounds or with a chiral smectic C liquid crystal composition.

For example, a strongly dielectric liquid crystal composition which is the mixture comprising:

| | |
|---|---|
| (S, S)-1-chloro-2-methylbutylcarboxylic acid 4-(4-octyloxyphenyl)phenyl ester | 39.0wt% |
| (S, S)-1-chloro-2-methylbutylcarboxylic acid 4-(4-nonyloxycarbonyloxyphenyl)phenyl ester | 22.0wt% |
| (S)-1-chloro-2-methylpropylcarboxylic acid 4-(4-nonyloxycarbonyloxyphenyl)phenyl ester | 20.0wt% |
| (S, S)-1-chloro-2-methylbutylcarboxylic acid 4-(4-nonylcarbonyloxyphenyl)phenyl ester | 19.0wt% |

exhibits a chiral smectic C phase only in a narrow temperature range of 38 to 60°C. A strongly dielectric liquid crystal composition which is the mixture of this composition with a compound of the present invention, namely 2-(4-nonyloxycarbonyloxyphenyl)-5-dodecylpyrimidine, in the ratio of 5:35, exhibits a chiral smectic C phase over a wide temperature range of room temperature to 63°C, and also has a very high response speed of 34 microseconds.

The present compounds are described in more detail below by Examples.

### EXAMPLE 1

### Synthesis and evaluation of 2-(4-decyloxyphenyl)-5-octyloxycarbonyloxypyrimidine (A)

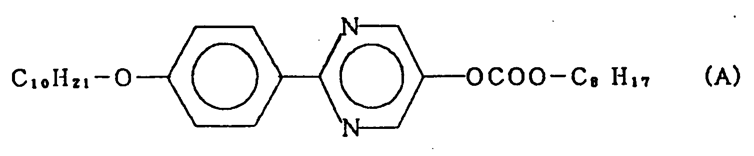

Ethyl benzyloxyacetate (B) was obtained by esterifying benzyloxyacetic acid as obtained from benzyl alcohol and chloroacetic acid by a conventional Williamson synthesis method. Compound (B) (9.7g) and 3.7g of ethyl formate were dissolved in 25ml of ether, and stirred in the presence of sodium for 2 hours, and ether was distilled off to obtain sodium 2-benzyloxy-2-ethoxycarbonylethylenolate (C). To this compound there was added a solution prepared by dissolving 14.2g of 4-decyloxybenzamidine hydrochloride (synthesized from 4-decyloxycyanobenzene by a conventional method) in 80ml of ethanol and by adding 1.2g of sodium, and the mixture was refluxed for 2 hours. The solvent was distilled off to obtain 2-(4-octyloxyphenyl)-4-hydroxy-5-benzyloxypyrimidine (D). Compound (D) (2g), together with phosphorous oxychloride, was refluxed for 3 hours to obtain 2-(4-octyloxyphenyl)-4-chloro-5-benzyloxypyrimidine (E).

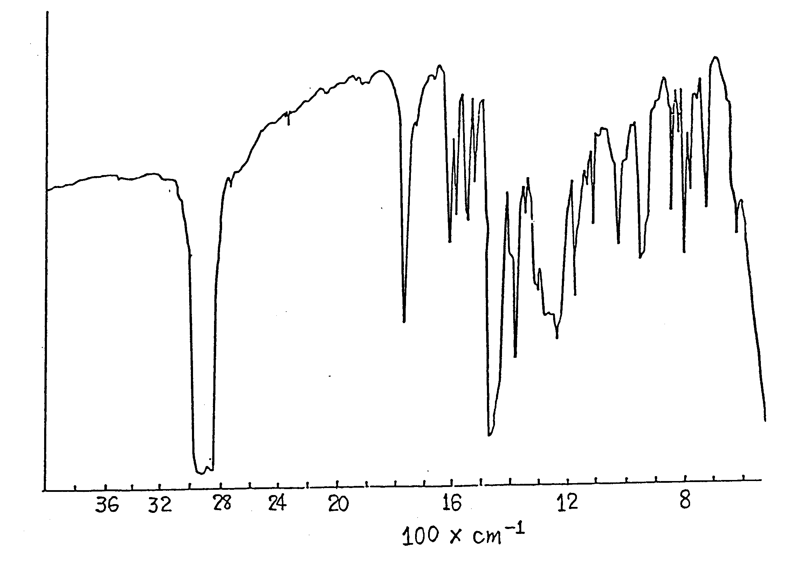

Compound (E) (1.7g) was hydrogenated in the presence of Pd-C and potassium carbonate in 1,4-dioxane at normal pressure to obtain 2-(4-octyloxyphenyl)-5-hydroxypyrimidine (F). Compound (F) (1.0g) was dissolved in 50ml of carbon tetrachloride and 0.8ml of octyl chlorocarboxylate and 5ml of pyridine were added. The mixture was stirred for a while and then allowed to stand overnight. 1N hydrochloride acid was added thereto, and the carbon tetrachloride layer was removed and concentrated. The resulting residue was purified by repeated recrystallization from methanol to obtain 2-(4-decyloxyphenyl)-5-octyloxycarbonyloxypyrimidine (A). The IR spectrum of this compound is shown in Figure 1. The phase transition point of this compound is given in Table 1.

### EXAMPLE 2

Various 2-(4-alkyloxyphenyl)-5-alkyloxycarbonyloxypyrimidines were synthesized from various 2-(4-alkyloxyphenyl)-5-hydroxypyrimidines as synthesized according to the method given in Example 1 and various chlorocarboxylic acid alkyl esters, according to the method given in Example 1. The phase transition points of the resulting compounds are given in Table 1.

### EXAMPLE 3

### Synthesis and evaluation of 2-(4-nonyloxycarbonyloxyphenyl)-5-dodecylpyrimidine (H)

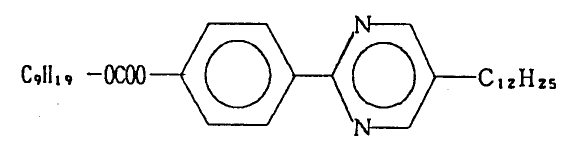

2-(4-Hydroxphenyl)-5-dodecylpyrimidine (G) (1.0g) was dissolved in 50ml of carbon tetrachloride, and 80ml of nonyl chlorocarboxylate and 5ml of pyridine were added. The mixture was stirred for a while and allowed to stand overnight. 1N hydrochloride acid was added, and the carbon tetrachloride layer was removed and concentrated. The residue was purified by repeated recrystallization from methanol to obtain compound (H).

### EXAMPLE 4

Various 2-(4-alkyloxycarbonyloxyphenyl)-5-alkylpyrimidine compounds were synthesized from various 2-(4-hydroxyphenyl)-5-alkylpyrimidine compounds and various chlorocarboxylic acid alkyl esters, according to the method as described in Example 3.

The phase transition points of the resulting compounds are given in Table 1.

### EXAMPLE 5

A chiral smectic C liquid crystal composition having the following composition was prepared:

| | |
|---|---|
| (S, S)-1-chloro-2-methylbutylcarboxylic acid 4-(4-octyloxyphenyl)phenyl ester | 25.4wt% |
| (S, S)-1-chloro-2-methylbutylcarboxylic acid 4-(4-nonyloxycarbonyloxyphenyl)phenyl ester | 14.3wt% |
| (S)-1-chloro-2-methylbutylpropylcarboxylic acid 4-(4-nonyloxycarbonyloxyphenyl)phenyl ester | 13.0wt% |
| (S, S)-1-chloro-2-methylbutylcarboxylic acid 4-(4-nonylcarbonyloxyphenyl)phenyl ester | 12.4wt% |
| 2-(4-nonyloxycarbonyloxyphenyl)-5-dodecylpyrimidine | 35.0wt% |

This composition exhibited a chiral smectic C phase at a temperature from room temperature to 63°C, a smectic A phase at a temperature from 63°C to 66°C and an isotropic phase at a temperature of 66°C or more.

The response speed of the composition, as determined by encapsulating it into a cell of 2.7 micrometres which had been subjected to an orientation treatment with a polyamide and exposing it to a rectangular wave of ±40V, was 34 microseconds at 25°C.

### EXAMPLE 6

A chiral smectic C liquid crystal composition having the following composition was prepared:

| | |
|---|---|
| (S, S)-1-chloro-2-methylbutylcarboxylic acid 4-(4octyloxyphenyl)phenyl ester | 26.5wt% |
| (S, S)-1-chloro-2-methylbutylcarboxylic acid 4-(4-nonyloxycarbonyloxyphenyl)phenyl ester | 15.0wt% |
| (S)-1-chloro-2-methylpropylcarboxylic acid 4-(4-nonyloxycarbonyloxyphenyl)phenyl ester | 13.6wt% |
| (S, S)-1-chloro-2-methylbutylcarboxylic acid 4-(4-nonylcarbonyloxyphenyl)phenyl ester | 12.9wt% |
| 2-(4-decyloxyphenyl)-5-octyloxycarbonyloxypyrimidine | 32.0wt% |

This composition exhibited a chiral smectic C phase at a temperature from room temperature to 58°C, a smectic A phase at a temperature from 58°C to 60°C, and an isotropic phase at a temperature of 60°C or more.

The response speed of the composition as determined by encapsulating it in a cell of 3.3 micrometres which had been subjected to an orientation treatment with a polyimide and exposing it to a rectangular wave of ±40V, was 20 microseconds at 25°C.

### EXAMPLE 7

A chiral smectic C liquid crystal composition having the following composition was prepared:

| | |
|---|---|
| (S, S)-1-chloro-2-methylbutylcarboxylic acid 4-(4-octyloxyphenyl)phenyl ester | 10.7wt% |
| (S, S)-1-chloro-2-methylbutylcarboxylic acid 4-(4-nonyloxycarbonyloxyphenyl)phenyl ester | 7.3wt% |
| (R)-4-(4-(4-nonyloxycarbonyloxyphenyl)benzoic acid 2-chloro-3-methylbutyl ester | 19.5wt% |
| 2-(4-nonyloxyphenyl)-5-nonylpyrimidine | 12.5wt% |
| 2-(4-octyloxyphenyl)-5-octylpyrimidine | 12.5wt% |
| 2-(4-octyloxyphenyl)-5-nonylpyrimidine | 12.5wt% |
| 2-(4-decyloxyphenyl)-5-octyloxycarbonyloxypyrimidine | 12.5wt% |
| 2-(4-nonyloxycarbonyloxyphenyl)-5-dodecylpyrimidine | 12.5wt% |

This composition exhibited a chiral smectic C phase at a temperature from below freezing point to 41°C, a smectic A phase at a temperature from 41°C to 61°C, and an isotropic phase at a temperature of 61°C or more.

The response speed of the composition, as determined by encapsulating it in a cell of 3.3 micrometres which had been subjected to an orientation treatment with a polyimide and exposing it to a rectangular wave of ±10V, was 24 microseconds at 23°C.

### EXAMPLE 8

A chiral smectic C liquid crystal composition having the following composition was prepared:

| | |
|---|---|
| (S, S)-1-chloro-2-methylbutylcarboxylic acid 4-(4-octyloxyphenyl)phenyl ester | 10.8wt% |
| (S, S)-1-chloro-2-methylbutylcarboxylic acid 4-(4-nonyloxycarbonyloxyphenyl)phenyl ester | 7.4wt% |
| (R)-4-(4-nonyloxycarbonyloxyphenyl)benzoic acid 2-chloro-3-methylbutyl ester | 20.0wt% |
| 2-(4-nonyloxyphenyl)-5-nonylpyrimidine | 12.7wt% |
| 2-(4-octyloxyphenyl)-5-octylpyrimidine | 12.7wt% |
| 2-(4-octyloxyphenyl)-5-nonylpyrimidine | 12.7wt% |
| 2-(4-decyloxyphenyl)-5-octyloxycarbonyloxypyrimidine | 6.6wt% |
| 2-(4-decyloxyphenyl)-5-pentyloxycarbonyloxypyrimidine | 3.9wt% |
| 2-(4-nonyloxycarbonyloxyphenyl)-5 heptylpyrimidine | 3.3wt% |
| 2-(4-nonyloxycarbonyloxyphenyl)-5dodecylpyrimidine | 4.9wt% |
| 2-(4-pentyloxycarbonyloxyphenyl)-5dodecylpyrimidine | 5.0wt% |

This composition exhibited a chiral smectic C phase at a temperature from below freezing point to 35°C, a smectic A phase at a temperature from 35°C to 59°C and an isotropic phase at a temperature of 59°C or more.

The response speed of the composition, as determined by encapsulating it in a cell of 3.3 micrometres which had been subjected to an orientation treatment with a polyimide and exposing it to a rectangular wave of ± 10V, was 18 microseconds at 23°C.

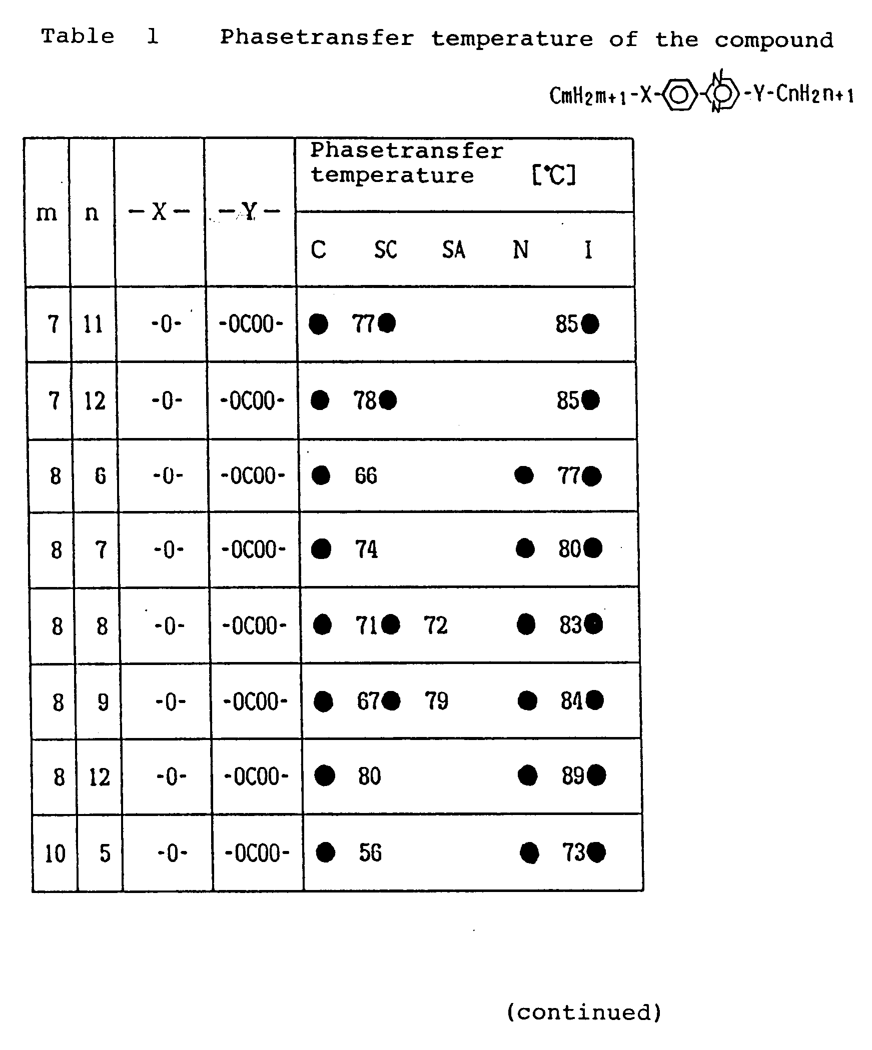

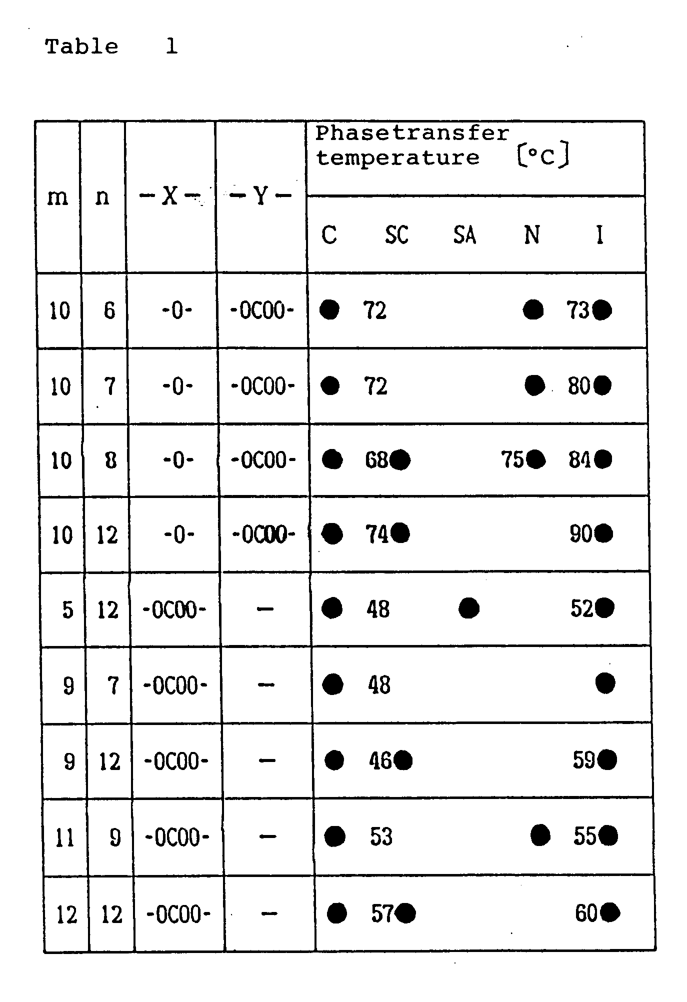

## Claims

1. A smectic liquid crystal compound represented by the general formula (I): 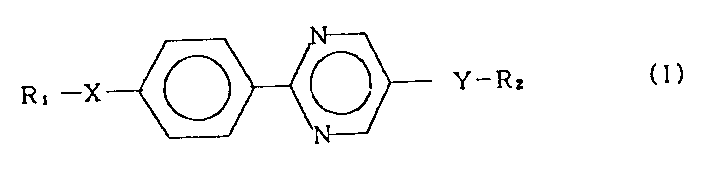 wherein R₁ and R₂ each represent an alkyl group having 1 to 18 carbon atoms, and X and Y each represent a single bond, -O-, -OCO-, -COO- or -OCOO-, provided that one of X and Y is -OCOO-.

2. A compound of claim 1 represented by the general formula (II): 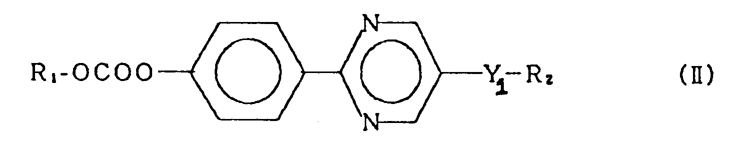 wherein R₁ and R₂ each represent an alkyl group having 1 to 18 carbon atoms, and Y₁ represents a single bond, -O-, -OCO- or -COO-.

3. A compound of claim 1 represented by the general formula (III): 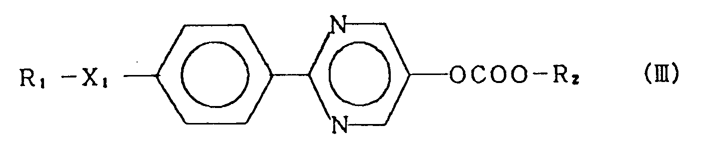 wherein R₁ and R₂ each represent an alkyl group having 1 to 18 carbon atoms, and X₁ represents a single bond, -O-, -OCO- or -COO-.

4. A chiral smectic C liquid crystal composition containing one or two or more of the smectic liquid crystal compounds claimed in any of claims 1 to 3.

## Patentansprüche

1. Smektische Flüssigkristall-Verbindung, welche durch die allgemeine Formel (I) dargestellt ist 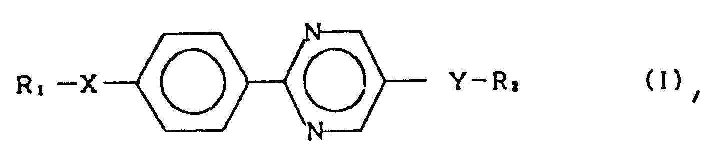 worin R₁ und R₂ jeweils eine Alkylgruppe mit 1 bis 18 Kohlenstoffatomen darstellen, und X und Y jeweils eine Einfachbindung, -O-, -OCO-, -COO- oder -OCOO- darstellen, mit der Maßgabe, daß eines der X und Y -OCOO- ist.

2. Verbindung gemäß Anspruch 1, welche durch die allgemeine Formel (II) dargestellt ist: 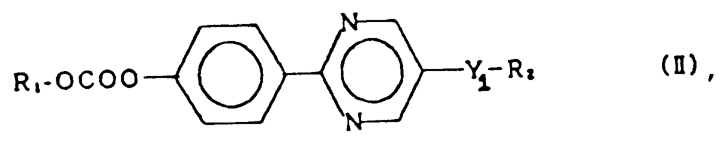 worin R₁ und R₂ jeweils eine Akylgruppe mit 1 bis 18 Kohlenstoffatomen darstellen, und Y₁ eine Einfachbindung, -O-, -OCO- oder -COO- darstellt.

3. Verbindung gemäß Anspruch 1, welche durch die allgemeine Formel (III) dargestellt ist: 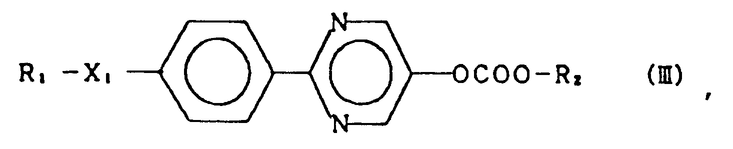 worin R₁ und R₂ jeweils eine Alkylgruppe mit 1 bis 18 Kohlenstoffatomen darstellen und X₁ eine Einfachbindung, -O-, -OCO-oder -COO- darstellt.

4. Chirale smektische C-Flüssigkristall-Zusammensetzung, welche eine, zwei oder mehr der in einem der Ansprüche 1 bis 3 beanspruchten smektischen Flüssigkristall-Verbindungen enthält.

## Revendications

1. Composé sous forme de cristaux liquides smectiques représenté par la formule générale (I): 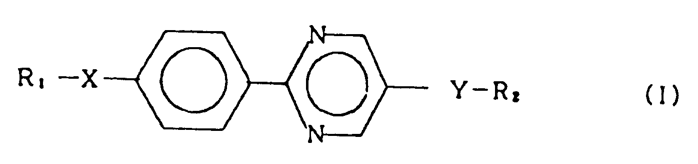 dans laquelle R₁ et R₂ représentent chacun un groupe alkyle ayant 1 à 18 atomes de carbone, et X et Y représentent chacun une liaison simple, -O-, -OCO-, -COO- ou -OCOO-, sous réserve que l'un des X et Y soit -OCOO-.

2. Composé selon la revendication 1, représenté par la formule générale (II): 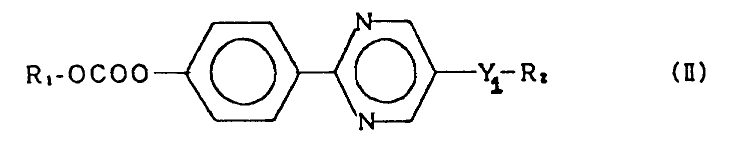 dans laquelle R₁ et R₂ représentent chacun un groupe alkyle ayant 1 à 18 atomes de carbone, et Y₁ représente une liaison simple, -O-, -OCO- ou -COO-.

3. Composé selon la revendication 1, représenté par la formule générale (III): 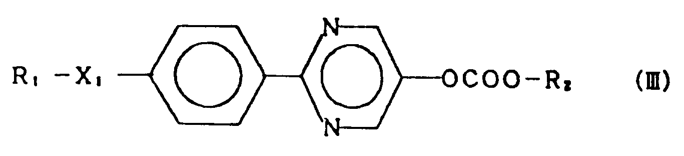 dans laquelle R₁ et R₂ représentent chacun un groupe alkyle ayant 1 à 18 atomes de carbone, et X₁ représente une liaison simple, -O-, -OCO- ou -COO-.

4. Composition de cristaux liquides chirale smectique C contenant un deux ou plusieurs des composés sous forme de cristaux liquides smectiques selon l'une quelconque des revendications 1 à 3.
